**Europäisches Patentamt**

**European Patent Office**

**Office européen des brevets**

(19)

(11) Veröffentlichungsnummer: **0 506 992 A1**

## (12) EUROPÄISCHE PATENTANMELDUNG

(21) Anmeldenummer: **91105281.9**

(22) Anmeldetag: **03.04.91**

(51) Int. Cl.5: **A61B 17/04**

(43) Veröffentlichungstag der Anmeldung:
**07.10.92 Patentblatt 92/41**

(84) Benannte Vertragsstaaten:
**AT CH ES FR GB IT LI NL SE**

(71) Anmelder: **Behrend, Detlef**
**He.-Aktivisten-Strasse 19**
**O-2530 Rostock-Warnemünde(DE)**
Anmelder: **Bartel, Frank**
**J-Nehru-Strasse 11**
**O-2540-Rostock 40(DE)**
Anmelder: **Schmitz, Klaus-Peter**
**Parkstrasse 39**
**O-2530 Rostock-Warnemünde(DE)**
Anmelder: **Reding, Richard**
**Kopernikusstrasse 38**
**O-2500 Rostock 1(DE)**

(72) Erfinder: **Behrend, Detlef**
**He.-Aktivisten-Strasse 19**
**O-2530 Rostock-Warnemünde(DE)**
Erfinder: **Bartel, Frank**
**J-Nehru-Strasse 11**
**O-2540-Rostock 40(DE)**
Erfinder: **Schmitz, Klaus-Peter**
**Parkstrasse 39**
**O-2530 Rostock-Warnemünde(DE)**
Erfinder: **Reding, Richard**
**Kopernikusstrasse 38**
**O-2500 Rostock 1(DE)**

(74) Vertreter: **Rother, Bernhard**
**Gdansker Strasse 5**
**O-2520 Rostock 22(DE)**

(54) **Mehrteilige Bauchwandverschlussplatte.**

(57) Bauchwandverschlußplattenteil, verstärkter Randbereich, Vorzugslöcher, Verschlußrand, Filzmaterial

Die Erfindung betrifft eine mehrteilige, elastische Bauchwandverschlußplatte, vorzugsweise aus thermoplastischen Polyurethanelastomeren (TPU), die es gestattet, einen temporären reversiblen Bauchwandverschluß, der beliebig oft wiederverschließbar ist, insbesondere bei der Oberbauchchirurgie, zu ermöglichen. Sie ist auch in der experimentiellen Chirurgie und Veterinärmedizin verwendbar.

Ziel und Aufgabe der Erfindung bestehen in der Entwicklung einer Verschlußplatte, insbesondere für die Bauchwand, die es gestattet, einerseits einen Spannungsarmen temporären Bauchverschluß bei adipösen Patienten durchzuführen, sowie andererseits eine wiederholte invasive Verlaufskontrolle ermöglicht.

Fig.1:

Die Erfindung betrifft eine mehrteilige, elastische Bauchwandverschlußplatte, vorzugsweise aus thermoplastischen Polyurethanelastomeren (TPU), die es gestattet, einen temporären reversiblen Bauchwandverschluß, der beliebig oft verschließbar ist, insbesondere bei der Oberhauchchirurgie, zu ermöglichen. Sie ist auch in der experimentiellen Chirurgie und Veterinärmedizin verwendbar.

Für den temporären Bauchverschluß werden in der Chirurgie z.Z. verschiedene Methoden angewendet. Vor allem zur Etappenlavagetherapie bei diffuser Peritonitis, nekrotisierender Pankreatitis und Mesenterialinfarkten verschließt man das Abdomenpanager mit Drahtstütznähten oder PVC-Schläuchen. Abgedichtet wird die Operationswunde mit Bauch- oder Gummitüchern. Als besonders nachteilig sind hierbei die starken Gewebereaktionen sowie die Nichtwiederverwendbarkeit der Nähte und der dadurch nicht gegebenen erneuten Einstichstellennutzung bei der erneuten Plazierung nach wiederholter OP-Sitzung. Zusätzlich kommt es bei septischen Operationen immer wieder zur Verschleppung von Keimen in die Muskulatur oder Bauchhaut mit der Gefahr der Ausbildung gefährlicher Bauchwandinfarkte.
Weiterhin wird von einigen Chirurgen zum temporären Bauchwandverschluß ein ETHISZIP-"Reißverschluß" (Firmenschrift der Fa. Ethicon Inc. USA) verwendet. Bei Anwendung dieses Systems treten jedoch massive Entzündungsreaktionen des Gewebes, insbesondere des Darmes, auf. Sie haben ihre Ursache hauptsächlich in mechanischenIrritationen wie Einklemmung des Gewebes beim Schließvorgang. Als wesentlicher Nachteil ist eine fehlende wirksame Sperre gegen das Eindringen von Mikroorganismen anzusehen.

Das Ziel der Erfindung besteht darin, daß durch die Verwendung einer mehrteiligen elastischen Bauchwandverschlußplatte dem Chirurgen eine invasive, komplikationsarme Verlaufskontrolle, insbesondere zur Etappenlavagetherapie, ermöglicht wird.

Der Erfindung liegt die Aufgabe zugrunde, eine Bauchwandverschlußplatte zu schaffen, die es dem Chirurgen ermöglicht, einerseits einen spannungsarmen temporären Bauchverschluß bei adipösen Patienten durchzuführen, sowie anderseits eine wiederholte invasive Verlaufskontrolle gestattet.
Die Aufgabe wird erfindungsgemäß dadurch gelöst, daß mehrere elastische Plattenteile vorzugsweise zwei mit verstärkten Randbereich und Vorzugslöchern zum Annähen durch den Chirurgen versehen ist und jedes Plattenteil über einen abgewinkelten Verschlußrand verfügt, der ebenfalls über Vorzugslöcher zum reversiblen Verschluß verfügt und weiterhin an der Trennfuge mit einem biokompatiblen chirurgischen Filzmaterial zur Verhinderung der Keimeinschleppung versehen ist.

Der Vorteil der mehrteiligen elastischen Bauchwandverschlußplatte besteht in einem sicheren und spannungsarmen Verschluß des oberen Abdomens sowie in der Möglichkeit diese beliebig oft zu öffnen und wieder zu verschließen.

Die Erfindung soll im folgenden an einem Ausführungsbeispiel näher erläutert werden, es zeigen:

Figur: 1    Aufsicht der zweiteiligen Bauchwandverschlußplatte

Figur: 2    Perspektvische Ansicht eines Bauchwandverschlußplattenteiles

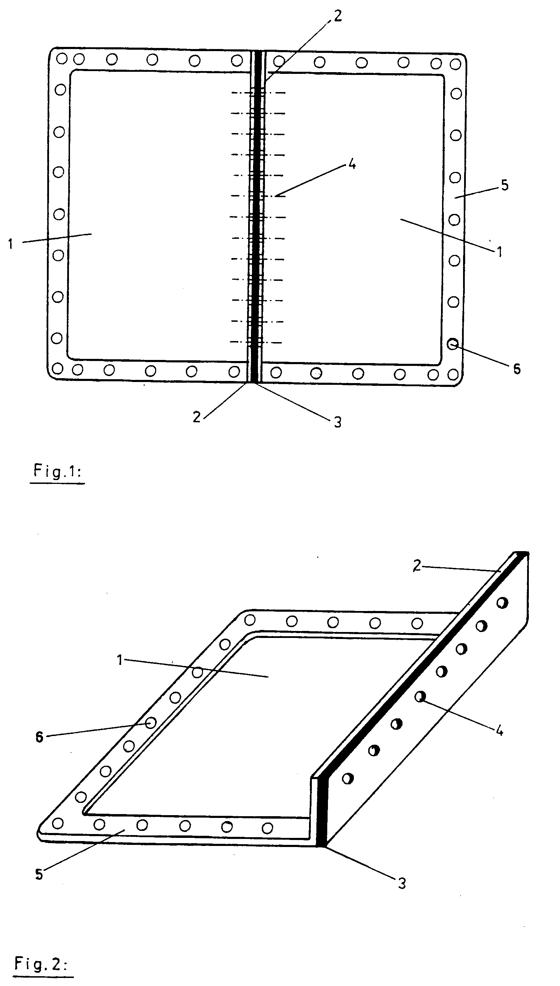

Gemäss Figur 1 befinden sich in den elastischen Bauchwandverschlußplattenteilen 1 Vorzugslöcher 4 in einem verstärkten Randhereich 5 , die das Annähen an die Bauchwand durch den Chirurgen ermöglichen. Die elastischen Bauchwandverschlußplattenteile 1 verfügen über je einen abgewinkelten Verschlußrand 2 , der zum Verbinden untereinander mit Vorzugslöchern oder Knopfelementen zum temporären Verschluß versehen ist. Figur 2 zeigt die perspektivische Ansicht eines Bauchwandverschlußplattenteiles 1 , dessen abgewinkelter Verschlußrand 2 an seiner Trennfuge mit einem biokompatiblen chirurgischen Filzmaterial 3 zur Verhinderung der Keimeinschleppung beschichtet ist.

**Patentansprüche**

1.    Mehrteilige Bauchwandverschlußplatte, gekennzeichnet dadurch, daß rechtes und linkes Bauchwandverschlußplatteteil (1) mittig einen abgewinkelten Verschlußrand (2) mit Vorzugslöchern oder Knopfelementen (4) sowie in der Trennfuge ein biokompatibles Filzmaterial (3) und einen verstärkten restlichen Randbereich (5) mit Vorzugslöchern (6) besitzt.

Fig.1:

Fig.2:

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 738 859 (ADAM)<br>* Abbildungen *<br>--- | 1 | A61B17/04 |
| A | US-A-4 210 148 (STIVALA)<br>* Zusammenfassung; Abbildungen *<br>--- | 1 | |
| A | US-A-3 648 705 (LARY)<br>* Abbildungen *<br>--- | 1 | |
| A | FR-A-2 013 707 (B. BRAUN KG)<br>--- | | |
| A | US-A-3 650 274 (EDWARDS ET AL.)<br>----- | | |
| | | | **RECHERCHIERTE SACHGEBIETE (Int. Cl.5)**<br><br>A61B<br>A61F |

Der vorliegende Recherchenbericht wurde für alle Patentansprüche erstellt

| Recherchenort | Abschlußdatum der Recherche | Prüfer |
|---|---|---|
| DEN HAAG | 13 NOVEMBER 1991 | SANCHEZ Y SANCHEZ J. |